# EUROPEAN PATENT SPECIFICATION

(11) **EP 2 473 218 B1**
(45) Date of publication and mention of the grant of the patent: **18.10.2017**
(21) Application number: 10747448.8
(22) Date of filing: 20.08.2010
(51) Int. Cl.: A61M 5/148, A61M 5/28, A61M 5/30, A61M 5/31, A61J 1/06

(54) **MEDICAMENT CONTAINER**
Arzneimittelbehälter
Conteneur de médicaments

(30) Priority: 02.09.2009 EP 09011255
(43) Date of publication of application: 11.07.2012
(73) Proprietor: Sanofi-Aventis Deutschland GmbH, 65929 Frankfurt am Main (DE)
(72) Inventor: NAGEL, Thomas, 01737 Tharandt (DE); RICHTER, René, 01737 Tharandt (DE); WITT, Robert, 01159 Dresden (DE)
(74) Representative: Finger, Catrin
(86) International application number: PCT/EP2010/062155
(87) International publication number: WO 2011/026738

(56) References cited:
- EP-A1- 2 042 207
- WO-A1-82/03556
- WO-A1-95/04561
- US-A- 2 805 662
- US-A1- 2001 016 710

## Description

### Technical Field

The invention relates to a medicament container, comprising a bag with an outlet, the bag compressible by a compression means.

### Background of the Invention

Many medicaments have to be injected into the body. This applies in particular to medicaments, which are deactivated or have their efficiency remarkably decreased by oral administration, e.g. proteines (such as insulin, growth hormones, interferons), carbohydrates (e.g. heparin), antibodies and the majority of vaccines. Such medicaments are predominantly injected by means of syringes, medicament pens or medicament pumps.

Some medicaments have to be administered by inhaling them from so called inhalers.

WO 2009/069518 A1 discloses an inhaler, wherein the medicament to be inhaled is stored in a bag shaped medicament container.

EP 20 42 207 A1 discloses a portable drug solution container that is filled with nearly one dose of throat drug solution, ensuring easy unsealing and use and realizing unsealing with the direction of nozzle fixed. There is provided a disposable portable drug solution container filled with a small amount of drug solution, including a platelike member capable of being folded at a given position into at least two segments and, bonded fast to the backside of the platelike member, a bag member filled with a drug solution, and further including a nozzle extending from a given position of the container, wherein in the state of having the platelike member folded, there is realized communication of the nozzle with the interior of the bag member.

US 2001/0016710 A1 discloses a drive mechanism for a medication delivery device includes a force receiving member, a force applying member and a shape memory alloy (SMA) actuator. The force applying member is operatively coupled to the force receiving member to move the force receiving member to a different position relative to the force applying member. The shape memory alloy actuator is formed from a shape memory alloy material and is operatively coupled to the force applying member. The shape memory alloy actuator is heat activated to distort the shape memory actuator from a first shape to a second shape to activate the force applying member to act upon the force receiving member to move the force receiving member to a different position relative to the force applying member.; Also, the shape memory alloy actuator is returned to the first shape from the second shape after the force receiving member is moved to a different position relative to the force applying member. In addition, the shape memory alloy actuator may be activated by applying and removing an electrical current to the shape memory element. For example, the drive mechanism may further include a power source coupled to the shape memory actuator to provide the electric current to the shape memory actuator. In addition, the shape memory actuator may be formed from Nitinol material, such as a wire. US 2001/016710 A1 discloses all the features of the preamble of claim 1.

US 2,805,662 discloses an arrangement for administering a liquid medicament comprising a medicament container with a bag, further comprising a compression means for compressing the bag, wherein the bag has an outlet and an inner surface. The initially open end of the medicament container may be welded after filling in the medicament. Re-expansion of the bag is avoided by the compression means gradually compressing the medicament container and keeping it compressed.

WO 82/03556 A1 discloses a wearable medicator suitable for controlled subcutaneous dispensing of a liquid medication into a patient. The medicator utilizes a unique arcuate syringe structure to provide a small compact medicator that can be easily worn by the patient. The medicator can provide controlled release of insulin for the treatment of diabetes.

WO 95/04561 A1 discloses means varying the contents within a receptacle and comprising a resilient member arranged to deform said receptacle so as to vary the contents therein. Said resilient member may be arranged so as to deform said receptacle so as to expel contents therefrom or introduce contents thereto. The resilient member may comprise a flat spring member arranged to wind from an unstable, uncoiled state towards a stable, coiled state and to thereby wind up said receptacle for expelling the contents from the receptacle. Valve control means can be associated with an opening in said receptacle so as to selectively control the discharge of the contents from said receptacle. Means may also be provided for charging said receptacle with contents to be subsequently discharged and formations may also be formed on said receptacle so as to vary the ease with which said spring member can wind said receptacle. During passages of movement of said spring member when said receptacle prevents a reduced resistance to motion thereof, said spring member can be arranged to drive a component of said apparatus, for example an extendable/retractable needle member.

### Summary of the Invention

It is an object of the present invention to provide an arrangement for administering a liquid medicament comprising a medicament container with a bag, further comprising a compression means for locally compressing the bag,wherein the bag has an outlet and an inner surface, wherein the arrangement avoids intake of air or reflow of remaining medicament into an emptied area of the bag when the compression means is pulled back from the bag after delivering a dose of medicament.

The object is achieved by an arrangement according to claim 1.

Preferred embodiments of the invention are given in the dependent claims. According to the invention an arrangement for administering the liquid medicament comprises a medicament container with a bag. Furthermore the arrangement comprises a compression means for locally compressing the bag. The medicament container according to the invention comprises a flexible bag with an outlet. The bag, preferably having a relatively thin skin, is compressible by the compression means so as to gradually reducing a volume of the bag and consequently squeezing the medicament stored in the bag out of the outlet similar to a tube of tooth paste. The bag is provided with an inner surface arranged to stick when subjected to the compression means. Thus the bag is kept from returning to its initial shape after having been subjected to the compression means so intake of air or reflow of the remaining medicament into the emptied area is avoided when the compression means is pulled back from the bag after delivering a dose of medicament. It is therefore not required to maintain the pressure exerted by the compression means after delivering a dose as opposed to conventional systems with rollers for compressing the bag and performing an advancing motion thereby sealing the portion of the bag already compressed against the portion not yet compressed.

In one embodiment of the invention the inner surface of the bag is self-adhesive so opposing surface parts stick together when brought in contact by the compression means.

In another embodiment of the invention the inner surface is arranged to be welded under pressure which may be exerted by the compression means,

In yet another embodiment of the invention the inner surface is arranged to be welded by heat. The heat may be introduced by a heated compression means.

The medicament container may be part of an injection arrangement or an inhaler arrangement for delivering a liquid medicament to a human or an animal, wherein the arrangement comprises the compression means for locally compressing the bag.
The injection arrangement may comprise a valve and a hollow needle for piercing a patient's skin, the valve and needle being arranged at the outlet of the medicament container. In case of a jet injector, instead of the needle, a jet nozzle may be arranged.

In a preferred embodiment of the invention the bag is arranged on an essentially planar, rigid support and a roller is arranged for locally pressing the bag against the support and advancing in a longitudinal direction of the bag. The roller may alternatively be replaced by a shoe or wiper or ram pressed against the bag.

The ram may be used to consecutively compressing, emptying and sealing a portion of the bag. The ram may be operated overlapping an edge of the bag in order to achieve delivery of a dose smaller than corresponding to an outline of the ram when placed in the middle of the bag.

The compression means may be arranged to introduce heat into the bag in order to achieve a heat-induced welding of the inner surface.

The injection arrangement may comprise a valve and a hollow needle for piercing a patient's skin, the valve and needle being arranged at the outlet of the medicament container. In case of a jet injector, instead of the needle, a jet nozzle may be arranged. The medicament container may preferably be used for delivering one of an analgetic, an anticoagulant, insulin, an insulin derivate, heparin, Lovenox, a vaccine, a growth hormone, a peptide hormone, a proteine, antibodies and complex carbohydrates.

Further scope of applicability of the present invention will become apparent from the detailed description given hereinafter. However, it should be understood that the detailed description and specific examples, while indicating preferred embodiments of the invention, are given by way of illustration only, since various changes and modifications within the scope of the invention as defined by the appended claims will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic view of an arrangement for administering a liquid medicament comprising a medicament container with a bag compressible by a compression means in the shape of a roller, and
- Figure 2: is a schematic view of another embodiment of the arrangement with the compression means in the shape of a ram.

Corresponding parts are marked with the same reference symbols in all figures. Detailed Description of Preferred Embodiments

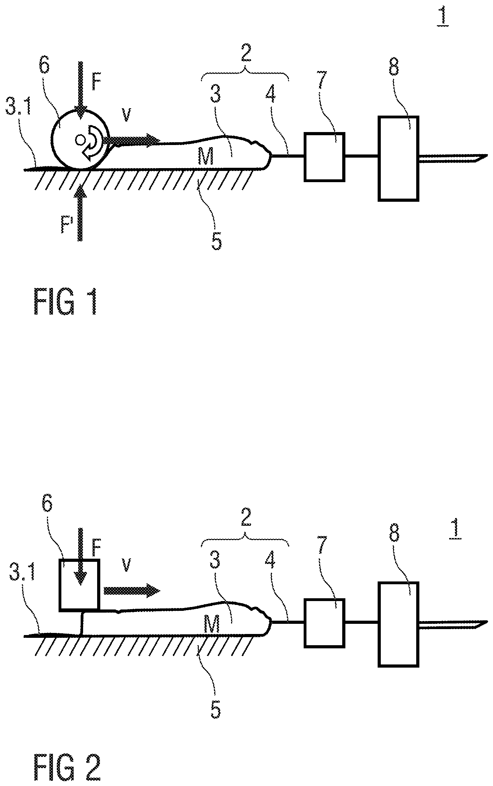

Figure 1 shows a schematic view of an arrangement 1 for administering a liquid medicament M comprising a medicament container 2. The medicament container 2 comprises a flexible bag 3 with an outlet 4. The bag 3 is arranged on an essentially planar, rigid support 5. The bag 3, preferably having a relatively thin skin, is compressible by a compression means 6 so as to gradually reducing a volume of the bag 3 and consequently squeezing the medicament M stored in the bag 3 out of the outlet 4. The bag 3 is provided with an inner surface arranged to stick when subjected to the compression means 6. When being compressed by the compression means 6, opposing parts of the inner surface are pressed together and consequently stick. Hence, after having been compressed by the compression means 6 the bag 3 is kept from returning to its initial shape in an already emptied portion 3.1.

The compression means 6 shown in figure 1 has the shape of a roller. The roller 6 is arranged for locally pressing the bag 3 against the support 5 with a force F while the support 5 exerts a counteracting force F'. In order to squeeze the medicament M out of the bag 3 the roller 6 is advanced in a longitudinal direction of the bag 3 with a speed v.

The inner surface of the bag 3 may be self-adhesive or arranged to be welded under pressure which may be exerted by the compression means 6.

The inner surface may also be arranged to be welded by heat. The heat may be introduced by a heated compression means 6.
The arrangement 1 shown in figure 1 is an injection arrangement comprising a valve 7 and a hollow needle 8 for piercing a patient's skin, the valve 7 and needle 8 being arranged at the outlet 4 of the medicament container 2. The arrangement 1 may alternatively be a jet injector with a jet nozzle instead of the needle 8.

The medicament container 2 may be part of another arrangement 1 for administering the liquid medicament M, e.g. an inhaler arrangement for delivering a liquid medicament M to a human or an animal.

Figure 2 is a schematic view of another embodiment of the arrangement 1 which differs from the embodiment shown in figure 1 by the compression means 6 having the shape of a ram. The ram 6 may be used to consecutively compressing, emptying and sealing a portion of the bag 3. Figure 2 shows the ram 6 being operated overlapping an edge of the bag 3 in order to achieve delivery of a dose smaller than corresponding to an outline of the ram when placed in the middle of the bag 3 or in another non-overlapping manner. The edge may be a lateral edge or an edge of the bag 3 towards the already emptied portion 3.1 as shown in figure 2.

The compression means 6 may also be a shoe or wiper pressed against the bag 3.

The medicament container 2 or the arrangement 1 may preferably be used for delivering one of an analgetic, an anticoagulant, insulin, an insulin derivate, heparin, Lovenox, a vaccine, a growth hormone, a peptide hormone, a proteine, antibodies and complex carbohydrates.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta-decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-SerSer-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(0)14 Trp(02)25, Asp28] Exendin-4(1-39),
   des Pro36 [Met(O)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39),
   wherein the group -Lys6-NH2 may be bound to the C-terminus of the Exendin-4 derivative;
or an Exendin-4 derivative of the sequence
   H-(Lys)6-des Pro36 [Asp28] Exendin-4(1-39)-Lys6-NH2,
   des Asp28 Pro36, Pyro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(02)25, Asp28] Exendin-4(1-39)-Lys6-NH2,
   H-des Asp28 Pro36, Pro37, Pro38 [Trp(02)25] Exendin-4(1-39)-NH2,
   H-(Lys)6-des Pro36, Pro37, Pro38 [Trp(02)25, Asp28] Exendin-4(1-39)-NH2,
   H-Asn-(Glu)5-des Pro36, Pro37, Pro38 [Trp(02)25, Asp28] Exendin-4(1-39)-NH2,
   des Pro36, Pro37, Pro38 [Trp(02)25, Asp28] Exendin-4(1-39)-(Lys)6-NH2,
   H-(Lys)6-des Pro36, Pro37, Pro38 [Trp(02)25, Asp28] Exendin-4(1-39)-(Lys)6-NH2,
   H-Asn-(Glu)5-des Pro36, Pro37, Pro38 [Trp(02)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(0)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [melt(O)14, Trp(02)25, Asp28] Exendin-4(1-39)-(Lys)6-NH2, H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pyro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leu-prorelin, Buserelin, Nafarelin, Goserelin.
A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

### List of References

- 1: arrangement for administering a liquid medicament
- 2: medicament container
- 3: flexible bag
- 3.1: already emptied portion
- 4: outlet
- 5: rigid support
- 6: compression means
- 7: valve
- 8: hollow needle
- F: force
- F': counteracting force
- M: medicament
- V: speed

## Claims

1. Arrangement (1) for administering a liquid medicament (M) comprising a medicament container (2) with a bag (3), further comprising a compression means (6) for locally compressing the bag (3), wherein the bag (3) has an outlet (4) and an inner surface, **characterized in that** the inner surface is arranged to stick when subjected to the compression means (6) and thus kept from returning to its initial shape after having been subjected to the compression means so intake of air or reflow of the remaining medicament (M) into the emptied area is avoided when the compression means (6) is pulled back from the bag (3) after delivering a dose of medicament.

2. Arrangement (1) according to claim 1, **characterized in that** the inner surface is self-adhesive.

3. Arrangement (1) according to one of the claims 1 or 2, **characterized in that** the inner surface is arranged to be welded under pressure.

4. Arrangement (1) according to one of the claims 1 to 3, **characterized in that** the inner surface is arranged to be welded by heat or by subjection to ultrasonic energy.

5. Arrangement (1) according to one of the claims 1 to 4, **characterized in that** the bag (3) is arranged on an essentially planar, rigid support (5).

6. Arrangement (1) according to one of the claims 1 to 4, **characterized in that** the bag (3) is arranged between two opposing parts of the compression means (6).

7. Arrangement (1) according to claim 5, **characterized in that** the compression means (6) is arranged for locally pressing the bag (3) against the support (5) and advancing at least in a longitudinal direction of the bag (3).

8. Arrangement (1) according to one of the claims 1 to 7, **characterized in that** the compression means (6) comprises at least one of a roller, a wiper, a shoe and a ram.

9. Arrangement (1) according to claim 6, **characterized in that** the compression means (6) comprises two rollers pressing against each other.

10. Arrangement (1) according to one of the claims 4 to 9, **characterized in that** the compression means (6) is arranged to introduce heat into the bag (3).

11. Arrangement (1) according to one of the claims 4 to 9, **characterized in that** the compression means (6) is arranged to introduce ultrasonic energy into the bag (3).

12. Arrangement (1) according to one of the claims 1 to 11, arranged to be an injection arrangement or an inhaler arrangement.

13. Arrangement (1) according to claim 12, **characterized in that** a valve (7) and a hollow needle (8) for piercing a patient's skin are arranged at the outlet (4).

## Patentansprüche

1. Anordnung (1) zur Verabreichung eines flüssigen Arzneimittels (M), umfassend einen Arzneimittelbehälter (2) mit einem Beutel (3), ferner umfassend ein Zusammendrückmittel (6) zum örtlichen Zusammendrücken des Beutels (3), wobei der Beutel (3) einen Auslass (4) und eine innere Fläche hat, **dadurch gekennzeichnet, dass** die innere Fläche so ausgebildet ist, dass sie haftet, wenn sie mit dem Zusammendrückmittel (6) beaufschlagt wird, und so von der Rückkehr zu ihrer ursprünglichen Form abgehalten wird, nachdem sie von dem Zusammendrückmittel beaufschlagt worden ist, so dass die Aufnahme von Luft oder das Rückströmen des restlichen Arzneimittels (M) in den geleerten Bereich vermieden wird, wenn das Zusammendrückmittel (6) nach Abgabe einer Arzneimitteldosis vom Beutel (3) zurückgezogen wird.

2. Anordnung (1) nach Anspruch 1, **dadurch gekennzeichnet, dass** die innere Fläche selbstklebend ist.

3. Anordnung (1) nach einem der Ansprüche 1 oder 2, **dadurch gekennzeichnet, dass** die innere Fläche zum Druckschweißen ausgebildet ist.

4. Anordnung (1) nach einem der Ansprüche 1 bis 3, **dadurch gekennzeichnet, dass** die innere Fläche für das Schweißen mittels Wärme oder mittels Beaufschlagung mit Ultraschallenergie ausgebildet ist.

5. Anordnung (1) nach einem der Ansprüche 1 bis 4, **dadurch gekennzeichnet, dass** der Beutel (3) auf einer im Wesentlichen planaren, starren Auflage (5) angeordnet ist.

6. Anordnung (1) nach einem der Ansprüche 1 bis 4, **dadurch gekennzeichnet, dass** der Beutel (3) zwischen zwei gegenüberliegenden Teilen des Zusammendrückmittels (6) angeordnet ist.

7. Anordnung (1) nach Anspruch 5, **dadurch gekennzeichnet, dass** das Zusammendrückmittel (6) so angeordnet ist, dass es den Beutel (3) örtlich gegen die Auflage (5) drückt und sich mindestens in einer Längsrichtung des Beutels (3) vorschiebt.

8. Anordnung (1) nach einem der Ansprüche 1 bis 7, **dadurch gekennzeichnet, dass** das Zusammendrückmittel (6) eine Rolle und/oder eine Rakel und/oder einen Schuh und/oder einen Stößel umfasst.

9. Anordnung (1) nach Anspruch 6, **dadurch gekennzeichnet, dass** das Zusammendrückmittel (6) zwei gegeneinander drückende Rollen umfasst.

10. Anordnung (1) nach einem der Ansprüche 4 bis 9, **dadurch gekennzeichnet, dass** das Zusammendrückmittel (6) so angeordnet ist, dass es Wärme in den Beutel (3) einführt.

11. Anordnung (1) nach einem der Ansprüche 4 bis 9, **dadurch gekennzeichnet, dass** das Zusammendrückmittel (6) so angeordnet ist, dass es Ultraschallenergie in den Beutel (3) einführt.

12. Anordnung (1) nach einem der Ansprüche 1 bis 11, ausgebildet als eine Injektionsanordnung oder eine Inhalatoranordnung.

13. Anordnung (1) nach Anspruch 12, **dadurch gekennzeichnet, dass** ein Ventil (7) und eine hohle Nadel (8) zum Einstechen in die Haut des Patienten am Auslass (4) angeordnet sind.

## Revendications

1. Agencement (1) pour administrer un médicament (M) comprenant un conteneur de médicament (2) présentant un sac (3), comprenant en outre des moyens de compression (6) pour comprimer localement le sac (3), dans lequel le sac (3) présente un sortie (4) et une surface intérieure, **caractérisé en ce que** la surface intérieure est agencée de manière à se coller lorsqu'elle est soumise aux moyens de compression (6) et est donc empêchée de reprendre sa forme initiale une fois qu'elle a été soumise aux moyens de compression de telle sorte qu'une entrée d'air ou un reflux du médicament restant (M) dans la zone vidée soit évité(e) lorsque les moyens de compression (6) sont retirés du sac (3) après l'administration d'une dose de médicament.

2. Agencement (1) selon la revendication 1, **caractérisé en ce que** la surface intérieure est auto- adhésive.

3. Agencement (1) selon l'une quelconque des revendications 1 ou 2, **caractérisé en ce que** la surface intérieure est agencée de manière à être soudée sous pression.

4. Agencement (1) selon l'une quelconque des revendications 1 à 3, **caractérisé en ce que** la surface intérieure est agencée de manière à être soudée par application de chaleur ou par soumission à une énergie ultrasonique.

5. Agencement (1) selon l'une quelconque des revendications 1 à 4, **caractérisé en ce que** le sac (3) est agencé sur un support essentiellement planaire et rigide (5).

6. Agencement (1) selon l'une quelconque des revendications 1 à 4, **caractérisé en ce que** le sac (3) est agencé entre deux parties opposées des moyens de compression (6).

7. Agencement (1) selon la revendication 5, **caractérisé en ce que** les moyens de compression (6) sont agencés de manière à presser localement le sac (3) contre le support (5) et à avancer au moins dans une direction longitudinale du sac (3).

8. Agencement (1) selon l'une quelconque des revendications 1 à 7, **caractérisé en ce que** les moyens de compression (6) comprennent au moins un élément parmi un rouleau, un balai, un patin et un poussoir.

9. Agencement (1) selon la revendication 6, **caractérisé en ce que** les moyens de compression (6) comprennent deux rouleaux qui exercent une pression l'un contre l'autre.

10. Agencement (1) selon l'une quelconque des revendications 4 à 9, **caractérisé en ce que** les moyens de compression (6) sont agencés de manière à introduire de la chaleur dans le sac (3).

11. Agencement (1) selon l'une quelconque des revendications 4 à 9, **caractérisé en ce que** les moyens de compression (6) sont agencés de manière à introduire une énergie ultrasonique dans le sac (3).

12. Agencement (1) selon l'une quelconque des revendications 1 à 11, agencé de manière à être un agencement d'injecteur ou un agencement d'inhalateur.

13. Agencement (1) selon la revendication 12, **caractérisé en ce qu'**une soupape (7) et une aiguille creuse (8) pour percer la peau d'un patient sont agencées à la sortie (4).
